Europäisches Patentamt

European Patent Office

Office européen des brevets

(11) Veröffentlichungsnummer: **0 308 841**

**A2**

(12) **EUROPÄISCHE PATENTANMELDUNG**

(21) Anmeldenummer: 88115293.8

(51) Int. Cl.⁴: **C07C 37/055 , C07C 39/15**

(22) Anmeldetag: **17.09.88**

(30) Priorität: 22.09.87 DE 3731794

(43) Veröffentlichungstag der Anmeldung:
**29.03.89 Patentblatt 89/13**

(84) Benannte Vertragsstaaten:
**CH DE FR GB IT LI**

(71) Anmelder: **BASF Aktiengesellschaft
Carl-Bosch-Strasse 38
D-6700 Ludwigshafen(DE)**

(72) Erfinder: **Stummp, Michael, Dr.
An der Marlach 13
D-6705 Deidesheim(DE)**
Erfinder: **Neumann, Peter, Dr.
Poststrasse 28
D-6800 Mannheim 31(DE)**
Erfinder: **Hahn, Erwin, Dr.
Am Buechsenackerhang 31
D-6900 Heidelberg(DE)**

(54) **Verfahren zur Herstellung von 4,4'-Dihydroxybiphenyl.**

(57) Verfahren zur Herstellung von 4,4'-Dihydroxybiphenyl durch Behandlung von 4,4'-Diacetylbiphenyl mit dem Peroxid einer Carbonsäure und anschließender Verseifung des bei der Oxidation entstandenen 4,4'-Diacetyloxybiphenyls.

EP 0 308 841 A2

## Verfahren zur Herstellung von 4,4'-Dihydroxybiphenyl

Die vorliegende Erfindung betrifft ein neues Verfahren zur Herstellung von 4,4'-Dihydroxybiphenyl durch Behandlung von 4,4'-Diacetylbiphenyl mit dem Peroxid einer Carbonsäure und anschließender Verseifung des bei der Oxidation entstandenen 4,4'-Diacetyloxybiphenyls.

4,4'-Dihydroxybiphenyl dient zur Herstellung von Polymeren, wie Polyurethanen, Polycarbonaten, Polyethern, Polyestern oder Polysulfonen. Derartige Polymere können zur Herstellung von hochtemperaturbeständigen Filmen, Fasern, Spritzgußteilen, extrudierten Körpern, Formkörpern oder Überzügen verwendet werden. In neuerer Zeit findet 4,4'-Dihydroxybiphenyl auch großes Interesse bei der Herstellung von Flüssigkristall-Polymeren.

Aus J. Chem. Soc. 1954, 2785, ist bekannt, 4,4'-Dihydroxybiphenyl durch oxidative Kupplung von unsubstituiertem Phenol herzustellen. Diese Reaktion ist jedoch wenig selektiv und verläuft in schlechten Ausbeuten.

Dagegen verläuft die Herstellung von 4,4'-Dihydroxybiphenyl durch oxidative Kupplung von sterisch gehinderten 2,6-Dialkylphenolen und anschließender Abspaltung der Alkylgruppen, wie sie z.B. in der US-A-4 482 755 beschrieben ist, selektiver und in besseren Ausbeuten. Die Ausübung dieses Verfahrens erfordert allerdings 2,6-Dialkylphenole als Ausgangsprodukte, die in technischem Maßstab nur schwer zugänglich sind.

Schließlich bedarf es zur Durchführung des in der DE-A-3 226 392 vorgeschlagenen Verfahrens zur Herstellung von 4,4'-Dihydroxybiphenyl durch selektive Sulfonierung von Biphenyl in para,para'-Position und anschließender Einführung der Hydroxylgruppen unter alkalischen Bedingungen eines erheblichen technischen Aufwands, da die Substitution der Sulfonsäuregruppen unter alkalischen Bedingungen bei einer Temperatur über 300 °C und teilweise unter erhöhtem Druck abläuft.

Aufgabe der vorliegenden Erfindung war nun die Bereitstellung eines selektiven Verfahrens zur Herstellung von isomerenreinem 4,4'-Dihydroxybiphenyl in guten Ausbeuten und ohne großen technischen Aufwand, wobei die Ausgangsprodukte leicht zugänglich sein sollten.

Es wurde gefunden, daß die Herstellung von 4,4'-Dihydroxybiphenyl in vorteilhafter Weise gelingt, wenn man 4,4'-Diacetylbiphenyl bei einer Temperatur von 30 bis 200 °C mit dem Peroxid einer Carbonsäure behandelt und anschließend das bei der Oxidation entstandene 4,4'-Diacetyloxybiphenyl in Gegenwart einer Base verseift.

Die Acylierung von Biphenyl mit Acetylchlorid oder Acetanhydrid zu 4,4'-Diacetylbiphenyl ist bekannt und beispielsweise in Agric. Biol. Chem., Vol. 37, 277 (1975), oder in Pharm. Ztg. Band 3, 352 (1966), beschrieben. Diese Umsetzung verläuft sehr selektiv, so daß das erhaltene 4,4'-Diacetylbiphenyl ohne aufwendige Reinigung im erfindungsgemäßen Verfahren verwendet werden kann.

Peroxide von Carbonsäuren sind z.B. Perameisensäure, Peressigsäure, Trifluorperessigsäure, Maleinmonopersäure, Perbenzoesäure, 3-Chlorperbenzoesäure, 4-Nitroperbenzoesäure, 4-Methoxycarbonylperbenzoesäure oder Phthalmonopersäure.

In der Regel verwendet man 2 bis 10 Mol, vorzugsweise 2 bis 6 Mol Peroxid, jeweils bezogen auf ein Mol 4,4'-Diacetylbiphenyl.

Die Behandlung mit Peroxid wird im allgemeinen in Gegenwart eines inerten organischen Verdünnungsmittels vorgenommen. Solche Verdünnungsmittel sind z.B. aliphatische, cycloaliphatische oder aromatische Kohlenwasserstoffe, die gegebenenfalls halogeniert, insbesondere chloriert sind, wie Ligroin, Petrolether, Cyclohexan, Benzol, Toluol, Xylol, Dichlormethan, Chloroform, 1,1,1-Trichlorethan, Chlorbenzol, Dichlorbenzol oder Trichlorbenzol.

Als Base zur Verseifung von 4,4'-Diacetyloxybiphenyl dienen Alkali- oder Erdalkalihydroxide oder -alkoholate insbesondere Natrium- oder Kaliumhydroxid oder Natrium- oder Kaliummethanolat, -ethanolat, -propanolat, -isopropanolat, -butanolat oder -isobutanolat.

Die Verwendung von Natrium- oder Kalium-$C_1$-$C_4$-alkanolat ist besonders bevorzugt.

Im allgemeinen kommen bei der Verseifung 2 bis 8 Mol Base, bezogen auf ein Mol 4,4'-Diacetyloxybiphenyl zur Anwendung.

Zweckmäßig wird das erfindungsgemäße Verfahren so durchgeführt, daß man 4,4'-Diacetylbiphenyl, Peroxid und Verdünnungsmittel vorlegt und unter Rühren auf eine Temperatur von 30 bis 200 °C, vorzugsweise 50 bis 100 °C erwärmt. In manchen Fällen kann es auch von Vorteil sein, nur einen Teil des Peroxids vorzulegen und während der Reaktion den restlichen Teil des Peroxids portionsweise zuzugeben. Danach wird für einen Zeitraum von 5 bis 20 Stunden bei der obengenannten Temperatur nachgerührt.

Die Isolierung des bei der Oxidation entstandenen 4,4'-Diacetyloxybiphenyls erfolgt dadurch, daß man das abgekühlte Reaktionsgemisch auf Wasser gibt, gegebenenfalls unter Zusatz eines Reduktionsmittels, z.B. Natriumsulfit, überschüssiges Peroxid zerstört, die organische Phase abtrennt und sie nach Waschen mit verdünnter Alkalicarbonatlösung einengt.

Das entstandene rohe 4,4'-Diacetyloxybiphenyl kann nun sofort der Verseifung unterworfen werden. Die Verseifung wird üblicherweise in einem Alkohol bei einer Temperatur von 60 bis 130° C vorgenommen. Verwendet man ein Alkoholat als Base, so setzt man zweckmäßig auch den entsprechenden Alkohol als Reaktionsmedium ein. Bei der Verwendung von Alkalihydroxiden empfiehlt es sich, zusätzlich in Gegenwart von Wasser zu arbeiten.

Nach Beendigung der Verseifung, die im allgemeinen 1 bis 10 Stunden in Anspruch nimmt, wird das Reaktionsgemisch abgekühlt, gegebenenfalls durch Zugabe von Säure neutral gestellt und das ausgefallene 4,4'-Dihydroxybiphenyl abgesaugt. Das Zielprodukt kann durch Umkristallisation aus Ethanol noch weiter gereinigt werden.

Das erfindungsgemäße Verfahren zeichnet sich durch seine milden Reaktionsbedingungen aus. Außerdem entsteht dabei 4,4'-Dihydroxybiphenyl in hoher Ausbeute, und es fallen nur wenig Nebenprodukte an.

Das folgende Beispiel soll die Erfindung näher beschreiben. Die dort genannten Prozente sind Gewichtsprozente.

Beispiel

7,14 g (0,03 Mol) 4,4'-Diacetylbiphenyl, 6 g (0,06 Mol) Natriumhydrogencarbonat und 25 g 3-Chlorperbenzoesäure wurden 15 Stunden in 150 ml Dichlormethan unter Rückfluß erhitzt. Nach Abkühlen auf Raumtemperatur wurde dann mehrmals mit 10%iger wäßriger Natriumhydrogensulfitlösung ausgerührt und die organische Phase mit wäßriger Kaliumcarbonatlösung ausgeschüttelt. Danach wurde die organische Phase mit Natriumsulfat getrocknet, abfiltriert und eingeengt. Man erhielt 6,9 g (85 % d.Th.) 4,4'-Diacetyloxybiphenyl.

Zur Verseifung wurde dieses Rohprodukt in 70 ml Ethanol mit 10 g Natriummethanolat 5 Stunden unter Rückfluß erhitzt. Danach wurde bei Raumtemperatur vom gebildeten Niederschlag abgesaugt und der Rückstand mehrmals mit Ethanol ausgewaschen. Die Filtrate wurden vereinigt, mit Aktivkohle versetzt, aufgekocht, abfiltriert und eingeengt. Man erhielt 4,3 g (91 % d.Th.) 4,4'-Dihydroxybiphenyl (Festpunkt: 278° C).

Ansprüche

1. Verfahren zur Herstellung von 4,4'-Dihydroxybiphenyl, dadurch gekennzeichnet, daß man 4,4'-Diacetylbiphenyl bei einer Temperatur von 30 bis 200° C mit dem Peroxid einer Carbonsäure behandelt und anschließend das bei der Oxidation entstandene 4,4'-Diacetyloxybiphenyl in Gegenwart einer Base verseift.

2. Verfahren gemäß Anspruch 1, dadurch gekennzeichnet, daß man 2 bis 10 Mol Peroxid je Mol 4,4'-Diacetylbiphenyl verwendet.

3. Verfahren gemäß Anspruch 1, dadurch gekennzeichnet, daß man die Verseifung von 4,4'-Diacetyloxybiphenyl mit einem Natrium- oder Kalium-$C_1$-$C_4$-alkanolat als Base vornimmt.